# EUROPEAN PATENT APPLICATION

(11) **EP 4 632 712 A1**
(43) Date of publication of application: **15.10.2025**
(21) Application number: 23902568.7
(22) Date of filing: 07.12.2023
(51) Int. Cl.: G08B 21/18, G01T 7/12

(54) **RADIATION SAFETY CONTROL SYSTEM FOR MEDICAL FACILITY**

(30) Priority: 16.12.2022 CN 202211622745
(71) Applicant: Neuboron Therapy System Ltd., Xiamen, Fujian 361026 (CN)
(72) Inventor: QIU, Honglin, Nanjing, Jiangsu 211112 (CN); WANG, Qian, Nanjing, Jiangsu 211112 (CN); LIU, Yuanhao, Nanjing, Jiangsu 211112 (CN)
(74) Representative: Gunzelmann, Rainer
(86) International application number: PCT/CN2023/136928
(87) International publication number: WO 2024/125372

(57) **Abstract**

The present invention relates to a radiation safety control system of a medical facility. The system includes: a radiation detection apparatus, which configured to collect an environmental radiation amount of each preset area in the medical facility; and an alarm apparatus, which performing corresponding alert and prompt based on the collected environmental radiation amount. The medical facility includes at least a first state, a second state, and a third state. When no radiation is generated in the medical facility, the medical facility is in the first state; when radiation is generated in at least one preset area in the medical facility, and an environmental radiation amount is less than a preset threshold, an area in which radiation is generated is in the second state, and another preset area is in the first state; and when an environmental radiation amount in at least one preset area is greater than or equal to the preset threshold, the medical facility is in the third state. **In** the present invention, relevant personnel in various areas in the facility can be notified of operating statuses of various devices in the medical facility timely and accurately, so that the relevant personnel perform corresponding operations.

## Description

### TECHNICAL FIELD

The present invention relates to the field of safety control technologies, and specifically, to a radiation safety control system of a medical facility.

### BACKGROUND

With the development of atomic science, radiation therapy such as Cobalt-60, linear accelerator, and electron beam has become one of the main means of cancer therapy. However, conventional photon or electron therapy is limited by physical conditions of the radiation. When neoplastic cells are killed, a large quantity of normal tissues in a beam path are also damaged. In addition, due to different degrees of sensitivity of neoplastic cells to radiation, the conventional radiation therapy usually has poor effects on radiation-resistant malignant neoplasms (such as Glioblastoma Multiforme and Melanoma).

To reduce radiation damage to normal tissues around the neoplasm, a concept of targeted therapy in chemotherapy is applied to the radiation therapy. Radiation sources, for example, proton therapy, heavy particle therapy, and neutron capture therapy, having a high relative biological effectiveness (RBE) are also actively developed currently for highly-radiation-resistant neoplastic cells. The neutron capture therapy combines the foregoing two concepts. For example, boron neutron capture therapy (BNCT for short), by means of specific aggregation of boron-containing medicine at the neoplastic cell in cooperation with precise neutron beam control, provides a selection of cancer therapy better than the conventional radiation therapy.

Conventional devices for proton therapy, devices for carbon ion therapy, and the like are mature. Several sets of facilities are put into operation domestically. The BNCT technology is a new therapy technology, but neutrons involved in the BNCT technology have radiation features different from those of particles such as protons and carbon ions. Therefore, the BNCT technology has higher radiation shielding difficulty and higher measurement difficulty. A commercial BNCT device needs an accelerator-based neutron source to provide a stable and controllable neutron beam. The neutron source is installed in a dedicated building facility having a radiation shielding capability. When the neutron source operates, radiation safety of all personnel in the facility needs to be ensured. Therefore, a matching safety control system is needed. However, a conventional safety control system cannot be directly applied to a BNCT facility in which a neutron beam is used as the therapy beam, and there is no safety control system applicable to the BNCT facility in the conventional technology either.

### SUMMARY

**In** view of this, it is necessary to provide, for the foregoing technical problem, a radiation safety control system of a medical facility applicable to a BNCT therapy facility and that has high safety.

According to a first aspect, the present invention provides a radiation safety control system of a medical facility. The medical facility includes a therapy system, and the therapy system includes a radiation generation unit and an irradiation unit. The radiation generation unit generates a radiation beam, and outputs the radiation beam by using the irradiation unit for irradiation. The radiation safety control system includes: a radiation detection apparatus, disposed in the medical facility, and configured to collect an environmental radiation amount of each preset area in the medical facility; an alarm apparatus, disposed in the medical facility, and performing corresponding alert and prompt based on the collected environmental radiation amount; and a server, connected to the radiation detection apparatus, and receiving a data value collected by the radiation detection apparatus. The server is connected to the alarm apparatus, and outputs an alarm instruction; and the server is connected to the therapy system, to control operating of the therapy system. The medical facility includes at least a first state, a second state, and a third state. When no radiation is generated in the medical facility, the medical facility is in the first state; when radiation is generated in at least one preset area in the medical facility, and an environmental radiation amount is less than a preset threshold, the medical facility is in the second state, and another preset area is in the first state; and when an environmental radiation amount in at least one preset area is greater than or equal to the preset threshold, the medical facility is in the third state.

In an embodiment, the preset area includes a therapy room and a therapy monitoring room corresponding to the therapy room; and the alarm apparatus includes:
a first alarm unit, disposed in the therapy room, and outputting alarm signals of at least two forms; and
a second alarm unit, disposed in the therapy monitoring room, and outputting alarm signals of at least three forms.

In an embodiment, when the medical facility is in the first state, both the first alarm unit and the second alarm unit send safe signals; when at least one therapy room is in the second state, a first alarm unit in the therapy room in the second state sends an evacuation signal, a second alarm unit in a corresponding therapy monitoring room sends an evacuation signal, and a second alarm unit in another therapy monitoring room sends an attention signal; and when the medical facility is in the third state, both the first alarm unit and the second alarm unit send evacuation signals.

In an embodiment, the preset area further includes a non-therapy area; the alarm apparatus includes a third alarm unit disposed in the non-therapy area and outputting alarm signals of at least two forms; and when the medical facility is in the first state, the third alarm unit sends a safe signal; when the medical facility is in the third state, the third alarm unit sends an evacuation signal; or when the medical facility is in the second state, the third alarm unit sends an attention signal indicating a status of the therapy room, and information of a therapy room currently performing beam emission can be learned based on the signal sent by the third alarm unit.

In an embodiment, the alarm apparatus includes an alarm light and/or a speaker for outputting different information.

In an embodiment, the preset area includes at least one therapy room; the speaker outputs a beam emission notification signal before the therapy room performs irradiation; and the speaker outputs a voice notification after the therapy room starts irradiation, to prompt personnel in the medical facility that the irradiation unit is operating.

In an embodiment, the therapy room, the therapy monitoring room, and the non-therapy area include at least one shielding space, a shielding door for shielding radiation is disposed in the shielding space, a fourth alarm unit is disposed on the shielding door, and the fourth alarm unit outputs alarm signals of at least three forms.

In an embodiment, when the medical facility is in the first state, the fourth alarm unit outputs a safe signal; when at least one of the shielding space is in the second state, a fourth alarm unit disposed on a shielding door in the shielding space in the second state outputs an attention signal; and when the medical facility is in the third state, the fourth alarm unit outputs an evacuation signal.

In an embodiment, the therapy system is a neutron capture therapy system, and the radiation detection apparatus detects an environmental radiation amount of neurons or γ rays in the medical facility.

In an embodiment, the radiation safety control system further includes a display device, and the display device is connected to the server, and is configured to display an operating parameter of the medical facility and an alarm status of the alarm apparatus.

According to a second aspect, the present invention further provides a control method for the foregoing radiation safety control system of a medical facility, including the following steps: detecting an environmental radiation amount in real time; comparing the environmental radiation amount detected in real time with a preset threshold in a server, and determining a state of the medical facility; and outputting, by the alarm apparatus, a signal based on the state of the medical facility.

In an embodiment, comparing the environmental radiation amount detected in real time with the threshold, when no radiation is generated in the medical facility, the medical facility is in a first state; when the environmental radiation amount is less than the threshold, and the medical facility includes at least one area in which radiation irradiation is performed, the area in which radiation irradiation is performed is in a second state; and when the environmental radiation amount is greater than or equal to the threshold, the medical facility is in a third state.

In an embodiment, when the medical facility is in the first state, an alarm unit in the medical facility sends a safe signal; an alarm unit in an area in the second state sends an evacuation signal, and an alarm unit in another area sends an attention signal; and when the medical facility is in the third state, an alarm unit in the medical facility sends an evacuation signal.

In the present invention, by disposing the radiation detection apparatus, the alarm apparatus, and the server, operating statuses of various devices in the medical facility can be notified to relevant personnel in various areas in the facility in time and accurately, so that the relevant personnel can learn a radiation safety condition of an environment in which the relevant personnel are located, and perform a corresponding operation. The radiation safety control system of the present invention uses a redundancy design, and for each operating status of the medical facility, a plurality of alarm units are used for alarm and notification in cooperation, thereby improving system reliability. Distinguishment is performed according to functions of areas in the facility and personnel responsibilities, so that standardization of normal operation of the system can be improved, and an occurrence rate of radiation safety accidents can be reduced.

### BRIEF DESCRIPTION OF THE DRAWINGS

FIG. 1 is a schematic diagram of a radiation safety control system according to an embodiment of the present invention;
FIG. 2 is a schematic structural diagram of a therapy system according to an embodiment of the present invention;
FIG. 3 is a schematic structural diagram of a first floor of a medical facility according to an embodiment of the present invention; and
FIG. 4 is a schematic structural diagram of a second floor of a medical facility according to an embodiment of the present invention.

### Descriptions of reference numerals:

1: Medical facility; 100: radiation safety control system; 200: therapy system; 101: therapy room; 101a: horizontal irradiation room; 101b: vertical irradiation room; 102: beam generation room; 102a: accelerator room; 103: therapy monitoring room; 110: shielding door;
10: radiation detection apparatus; 20: alarm apparatus; 30: server; 21: first alarm unit; 22: second alarm unit; 23: third alarm unit; 21a, 21b, and 21c: first alarm lights; 22a, 22b, 22c: second alarm lights; 23a, 23b, 23c, 23d, 23e, 23f, and 23g: third alarm lights; 23h, 23i, 23j, 23k, and 23l: fourth alarm lights; 31: client host; 32: server host; 33: display device;
210: radiation generation unit; 220: irradiation unit; 230: positioning unit; 211: accelerator; 221: beam shaping assembly; 222: beam exit; 2211: reflector; 2212: moderator; 2213: thermal neutron absorber; 2214: radiation shield; 2215: beam channel.

### DETAILED DESCRIPTION

To make the objectives, technical solutions, and advantages of this application clearer, the following further describes this application in detail with reference to the accompanying drawings and the embodiments. The specific embodiments described herein are only used to explain this application, and are not intended to limit this application.

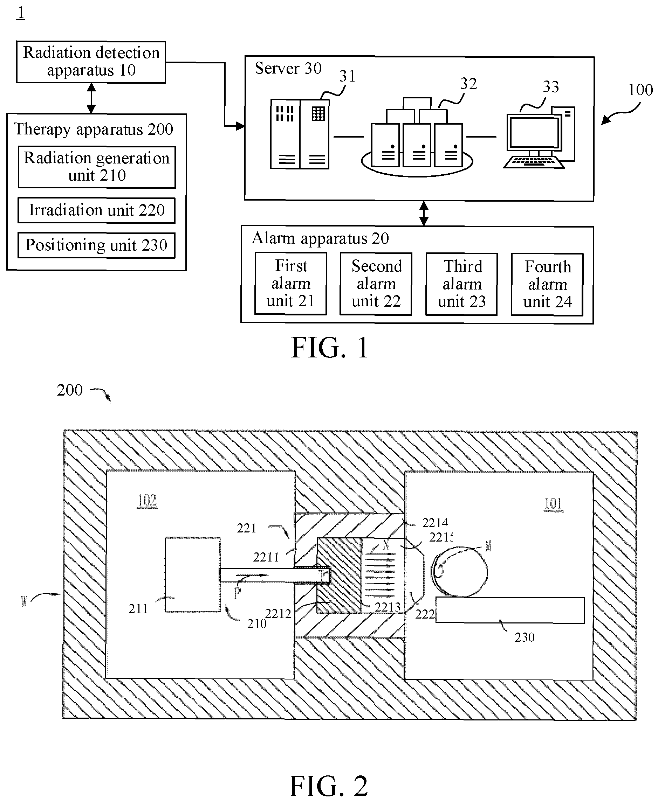

Referring to FIG. 1, FIG. 1 shows a schematic structural diagram of a radiation safety control system 100 disposed in a medical facility 1. The radiation safety control system 100 includes a radiation detection apparatus 10, an alarm apparatus 20, and a server 30. A therapy system 200 is further disposed in the medical facility 1. The therapy system 200 includes at least a radiation generation unit 210, an irradiation unit 220, a positioning unit 230, and a therapy planning unit. In this embodiment, the therapy system 200 is a boron neutron capture therapy system. The radiation detection apparatus 10 is disposed in the medical facility 1, and is configured to collect an environmental radiation amount of each preset area in the medical facility. When the therapy system 200 is the boron neutron capture therapy system, the radiation detection apparatus detects an environmental radiation amount of neurons or γ rays in the medical facility 1. The alarm apparatus 20 is disposed in the medical facility 1, and performs corresponding alert and prompt based on the collected environmental radiation amount. The server 30 is connected to the radiation detection apparatus 10, and receives a data value collected by the radiation detection apparatus 10. The server 30 is connected to the alarm apparatus 20, and outputs an alarm instruction. The server 30 is connected to the therapy system 200, and controls operating of the therapy system 200.

With reference to FIG. 2, the radiation generation unit 210 is configured to generate a therapy neutron beam N. The therapy planning unit performs dose simulation calculation based on medical image data of an irradiated position and generates an initial therapy plan. The therapy plan includes an irradiation dose, an irradiation position, an irradiation angle, irradiation time, and the like. After a to-be-irradiated body is positioned at the irradiation position determined in the therapy plan, the irradiation unit 220 performs irradiation therapy according to the therapy plan. The positioning unit 230 is configured to accommodate the to-be-irradiated body, to change a position of the to-be-irradiated body, so that the to-be-irradiated body stably receives the neutron beam N, and the positioning unit can automatically or passively adjust the position of the to-be-irradiated body. Further, the positioning unit 230 may be a therapy bed, a therapy chair, or the like. An adjustment mechanism, such as a mechanical arm configured to automatically adjust a position of the positioning unit 230, may be connected to the positioning unit 230.

A main principle of the boron neutron capture therapy is as follows: After a to-be-irradiated body S takes or is injected with boron (^{B-}10)-containing medicine, the boron-containing medicine selectively accumulates in neoplastic cells. Then, because the boron (^{B-}10)-containing medicine features in high capture cross section for thermal neurons, two heavy charged particles: ⁴He and ⁷Li are generated through ¹⁰B(n, α)⁷Li neutron capture and nuclear fission reaction. Average energy of the two heavy charged particles is approximately 2.33 MeV, and the two particles have features of high linear energy transfer (LET) and short ranges, where a total range of the two particles is approximately equivalent to a size of a cell. Therefore, radiation damage caused to a living body can be limited to a cell level, and this can achieve an objective of locally killing neoplastic cells without causing too much damage to normal tissues.

As shown in FIG. 2, the radiation generation unit 210 includes an accelerator 211, a beam transmission apparatus, and a target T. The accelerator 211 accelerates charged particles (such as protons and deuterons), to generate a charged particle ray P such as a proton ray. The charged particle ray P is irradiated on the target T and reacts with the target T to generate a neutron ray (neutron beam) N. The target T is preferably a metal target. A suitable nuclear reaction is selected based on features such as a required neutron production rate and energy, energy and a current of accelerated charged particles that can be provided, and physicochemical properties of the metal target. The nuclear reaction that is usually discussed includes ⁷Li(p, n)⁷Be and ⁹Be(p, n)⁹B, where both the reactions are endothermic reactions. Energy thresholds of the two nuclear reactions are respectively 1.881 MeV and 2.055 MeV. Because an ideal neutron source for the boron neutron capture therapy is an epithermal neutron with an energy level of keV, theoretically, if a lithium metal target is bombarded by a proton with an energy only slightly higher than the threshold, a low-energy neutron may be generated, and the low-energy neutron can be used in clinic without much deceleration processing. However, two targets: lithium metal (Li) and beryllium metal (Be) has low reaction cross section with a proton at threshold energy. To generate a sufficiently large neutron flux, generally, a proton with high energy is selected for triggering the nuclear reaction. An ideal target should have features such as having a high neutron production rate, generating a neuron with energy distribution close to an epithermal neutron energy area, not generating excessively much penetrating radiation, being safe, cheap, and easy to operate, and high-temperature resistance. However, actually, a nuclear reaction satisfying all the requirements cannot be found. A target made of lithium metal is preferably used in the embodiments of the present invention. However, as well-known by a person skilled in the art, the material of the target T may alternatively be a metal material other than lithium or berylum, for example, may be tantalum (Ta) or tungsten (W). The target T may be in a circular plate shape, or may be in another solid shape, or may be a liquid (liquid metal). The accelerator 211 may be a linear accelerator, a cyclotron, a synchrotron, or a synchrocyclotron. Further, the radiation generation unit 210 may be a nuclear reactor rather than an accelerator and a target.

Regardless of whether the neutron source of the boron neutron capture therapy is from a nuclear reactor or from a nuclear reaction between a charged particle in an accelerator and a target, a generated radiation field is actually a hybrid radiation field, in other words, a beam includes low-energy to high-energy neutrons and photons. For the boron neutron capture therapy of deep neoplasm, a larger quantity of radiant rays other than epithermal neurons causes a larger proportion of non-selective dose deposition of normal tissues. Therefore, the quantity of the radiant rays that cause unnecessary dose deposition should be reduced as much as possible. The irradiation unit 220 is configured to adjust beam quality of the neutron beam generated by the radiation generation unit 210 and perform irradiation, to reduce unnecessary dose deposition and converge the neutron beam, so that the neutron beam has high targetability in a therapy process.

Specifically, the irradiation unit 220 includes a beam shaping assembly 221 configured to adjust the beam quality of the neutron beam and a beam exit 222 configured to converge the neutron beam. The neutron beam N generated by the radiation generation unit 210 is irradiated to the to-be-irradiated body on the positioning unit 230 sequentially through the beam shaping assembly 221 and the beam exit 222. The beam shaping assembly 221 can adjust the beam quality of the neutron beam N generated by the radiation generation unit 210. The beam exit 222 may be an exit provided with a collimator, and is configured to converge the neutron beam N, so that the neutron beam N has high targetability in the therapy process. It may be understood that, a collimator may alternatively be not included in this embodiment, and after exiting from the beam shaping assembly 221, the beam is directly irradiated to the to-be-irradiated body on the positioning unit 230.

The beam shaping assembly 221 further includes a reflector 2211, a moderator 2212, a thermal neutron absorber 2213, a radiation shield 2214, and a beam channel 2215. Because the neutron generated by the radiation generation unit 210 has a very wide energy spectrum, other than epithermal neurons satisfying a therapy requirement, quantities of other types of neutrons and photons need to be reduced as much as possible, to avoid damage to operating personnel or the to-be-irradiated body. Therefore, a neutron exiting from the target T needs to pass through the moderator 2212, to adjust fast neutron energy (> 40 keV) of the neutron to an epithermal neutron energy area (0.5 eV to 40 keV), and reduce thermal neutrons (< 0.5 eV) as much as possible. The moderator 2212 is made of a material having a large reaction cross section with the fast neutron and a small reaction cross section with the epithermal neutron. In a preferred embodiment, the moderator 2212 is made of at least one of D₂O, AlF₃, Fluental^{™}, CaF₂, Li₂CO₃, MgF₂, and Al₂O₃. The reflector 2211 surrounds the moderator 2212, and reflects, back to the neutron beam N, neutrons diffusing after passing through the moderator 2212, to improve neutron utilization, and the reflector is made of a material having a strong neutron reflecting capability. In a preferred embodiment, the reflector 2211 is made of at least one of Pb or Ni. There is a thermal neutron absorber 2213 at a rear portion of the moderator 2212, and the thermal neutron absorber 2213 is made of a material having a large reaction cross section with the thermal neutron. In a preferred embodiment, the thermal neutron absorber 2213 is made of Li-6. The thermal neutron absorber 2221 is configured to absorb thermal neutrons passing through the moderator 2212, to reduce a quantity of thermal neutrons in the neutron beam N, and prevent causing an excessive dose for superficial normal tissues. It may be understood that, the thermal neutron absorber 2213 may alternatively be integrated with the moderator, and a material of the moderator includes Li-6. The radiation shield 2214 is configured to shield neutrons and photons leaking from a part other than the beam channel 2215, and a material of the radiation shield 2214 includes at least one of a photon shielding material and a neutron shielding material. In a preferred embodiment, the material of the radiation shield 2214 includes a photon shielding material: lead (Pb), and a neutron shielding material: polyethylene (PE). The beam exit 222 is disposed at a rear portion of the beam channel 2215. An epithermal neutron beam exiting from the beam exit 222 is irradiated to the to-be-irradiated body, and after passing through superficial normal tissues, the epithermal neutron beam is decelerated to thermal neutrons, and reaches neoplastic cells in an affected part M.

It may be understood that the beam shaping assembly 221 may alternatively have another construction, provided that the beam shaping assembly 221 can obtain an epithermal neutron beam required for therapy. For ease of description, when no collimator is provided, the beam exit 222 is an exit of the beam channel 2215; when a collimator is provided, the beam exit 222 is an exit of the collimator.

The medical facility 1 further includes a therapy room 101 and a beam generation room 102. The to-be-irradiated body 200 on the positioning unit 230 receives a therapy of neutron beam N irradiation in the therapy room 101. The beam generation room 102 at least partially accommodates the accelerator 111 and the beam transmission apparatus. The beam shaping assembly 221 is at least partially accommodated in a partition wall between the therapy room 101 and the beam generation room 102. It may be understood that the partition wall may completely separate the therapy room 101 and the beam generation room 102. Alternatively, may partially separate the therapy room 101 and the beam generation room 102, and the therapy room 101 communicates with the beam generation room 102. There may be one or more targets T. The charged particle ray P optionally interacts with one or more targets T, or interacts with a plurality of targets T at the same time, to generate one or more therapy neutron beams N. In correspondence to the quantity of the targets T, there may also be one or more beam shaping assemblies 221, beam exits 222, and positioning units 230. A plurality of positioning units may be disposed in a same therapy room, or an independent therapy room may be disposed for each positioning unit. The therapy room 101 and the beam generation room 102 are spaces surrounded by a concrete wall W (including the partition wall). The concrete structure can shield neutrons and other radiant rays leaked in an operating process of the radiation therapy system 200.

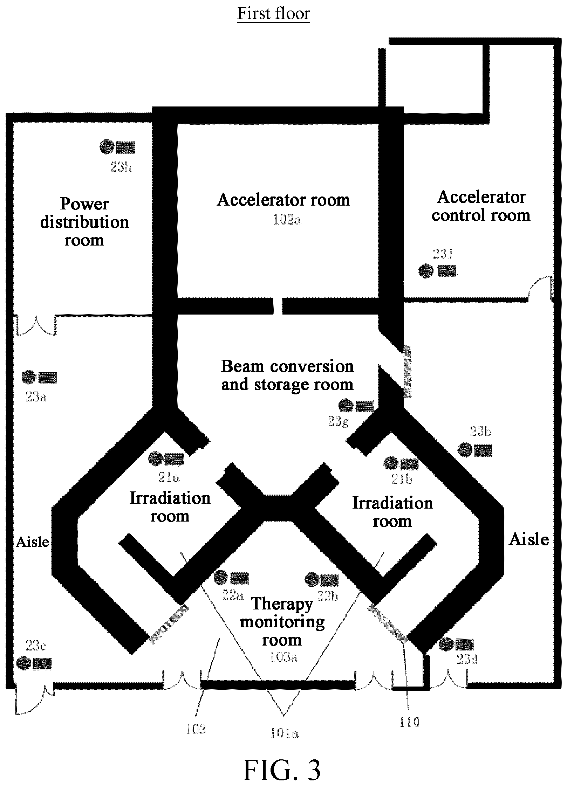

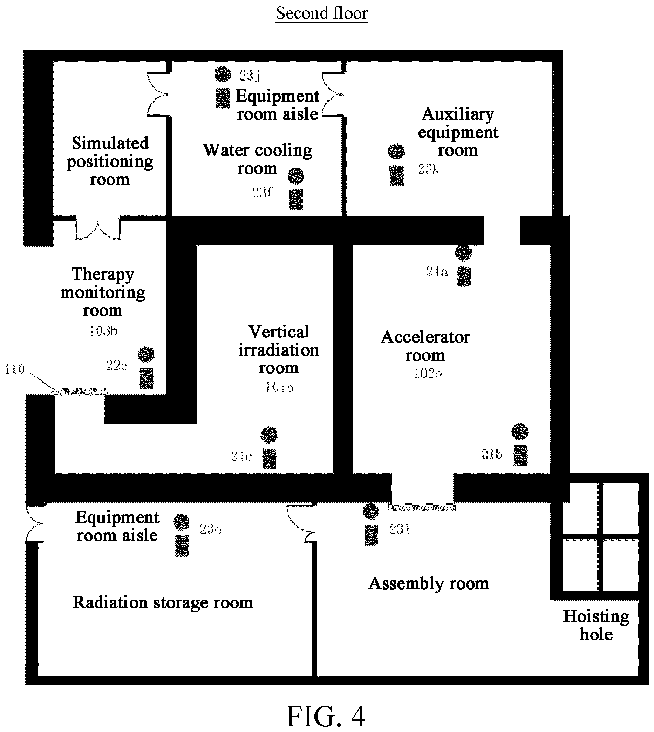

In this embodiment, the medical facility 1 includes the radiation safety control system 100, the therapy system 200, a building accommodating the radiation safety control system 100 and the therapy system 200, and a spatial range covering the radiation safety control system 100, the therapy system 200, and the foregoing building. One of the foregoing manners is used as an example for description in this embodiment. The preset area included in the medical facility 1 includes several independent rooms. A spatial range disposed in the medical facility 1 includes a therapy room, a therapy monitoring room, and a non-therapy area. In this embodiment disclosed in the present invention, the non-therapy area includes areas such as an aisle, an accelerator room, an accelerator control room, a power distribution room, a beam conversion and storage room, a simulated positioning room, a water cooling room, and an auxiliary equipment room. In this embodiment, the medical facility 1 includes a building having at least two floors. The therapy room 101, the therapy monitoring room 103, and the non-therapy area are distributed in different rooms on the floors. The therapy monitoring room 103 is configured to monitor an irradiation process in the therapy room 101. As shown in FIG. 3 and FIG. 4, a first-floor area and a second-floor area are shown in this embodiment.

The therapy room 101 includes a horizontal irradiation room 101a and a vertical irradiation room 101b, and the horizontal irradiation room 101a includes two irradiation rooms. The horizontal irradiation room 101a receives a neutron beam emitted in a horizontal direction, and is correspondingly provided with a first therapy monitoring room 103a. The vertical irradiation room 101b receives a neutron beam emitted in a vertical direction, and is correspondingly provided with a second therapy monitoring room 103b. The horizontal irradiation room 101a and the vertical irradiation room 101b are disposed on different floors in the medical facility 1, or are disposed at different heights. The therapy monitoring room 103 is configured to monitor various processes of the radiation therapy, and is generally disposed near the therapy room 101.

The beam generation room 102 includes an accelerator room 102a. The accelerator room 102a includes at least two accelerator rooms respectively disposed on different floors or at different heights in the medical facility 1, to cooperate with corresponding irradiation rooms.

The alarm apparatus 20 includes a first alarm unit 21, a second alarm unit 22, and a third alarm unit 23. The first alarm unit 21 is disposed in the therapy room, and outputs alarm signals of at least three forms. The second alarm unit 22 is disposed in the therapy monitoring room, and outputs alarm signals of at least three forms. The third alarm unit 23 is disposed in the non-therapy area, and outputs alarm signals of at least three forms. The alarm apparatus 20 may be disposed on a wall inside or outside a room according to specific uses of different rooms, and can instruct personnel in the room to evacuate, or prompt a person outside the room to stay away.

The medical facility 1 includes at least a first state, a second state, and a third state. When no radiation is generated in the medical facility 1, that is, no environmental radiation amount is detected in all areas in the medical facility 1, the medical facility 1 is in the first state. When radiation irradiation is performed in at least one preset area in the medical facility 1, and an environmental radiation amount is less than a preset threshold, the medical facility 1 is in the second state. When an environmental radiation amount in at least one preset area is greater than or equal to the preset threshold, the medical facility 1 is in the third state. Specifically, in this embodiment, the first state may be a standby safe state, which indicates that the therapy system 200 is not operating at this time. The second state is a beam-emitting state, which indicates that the therapy system 200 is operating at this time and the environmental radiation amount is less than the preset threshold at this time. The third state is an abnormal state, which indicates that the environmental radiation amount is greater than or equal to the preset threshold at this time.

When the medical facility 1 is in the first state, the first alarm unit 21, the second alarm unit 22, and the third alarm unit 23 all send first signals. When the medical facility 1 is in the second state, a first alarm unit 21 in a therapy room that is radiating a beam sends a third signal, and a second alarm unit 22 in a corresponding therapy monitoring room sends a third signal; a first alarm unit 21 in another therapy room sends a second signal, and a second alarm unit 22 in another corresponding therapy monitoring room sends a second signal; and a part of third alarm units 23 send second signals. When the medical facility 1 is in the third state, the first alarm unit 21, the second alarm unit 22, and the third alarm unit 23 all send third signals. Further, the first signal is a safe signal, the second signal is an attention signal, and the third signal is an evacuation signal.

In this embodiment, in three alarm signals outputted by the first alarm unit 21, at least two signals are different signals of a same type. In another embodiment, the three alarm signals may all be different types of signals, but there are obvious differences. This is not limited herein. As shown in FIG. 4, using the second-floor accelerator room 102a as an example, the first alarm unit 21 includes first alarm lights 21a, 21b, and 21c disposed in a plurality of rooms in the therapy room. The first alarm lights 21a, 21b, and 21c may output light of two colors as two forms of alarm signals, for example, red and green. That the green light is always-on indicates that the medical facility 1 is currently in a standby safe state. That the red light is always-on indicates that a current therapy room 101 is currently in a beam-emitting state, non-patient personnel needs to evacuate from the current therapy room 101 and another person is not allowed to enter again, and a person in another therapy room 101 needs to pay attention to the therapy room 101 in the beam-emitting state. Further, in addition to the foregoing two types of alarm signals, when the red light and the green light both flicker, it is used as the third type of alarm signal in this application. Flickering of the red light and the green light may indicate that the medical facility 1 is in an abnormal state, and all personnel need to evacuate. In another embodiment, the first alarm unit 21 further includes a speaker, configured to broadcast content of the alarm signal, where the content of the alarm signal includes content for indicating safe or evacuation.

In this embodiment, three forms of alarm signals outputted by the second alarm unit 22 are different signals of a same type. In another embodiment, the three forms of alarm signals may alternatively be different types of signals, provided that there are apparently differences. This is not limited herein. As shown in FIG. 3, using the therapy monitoring room 103 in this embodiment as an example, the therapy monitoring room 103 includes a first therapy monitoring room 103a and a second therapy monitoring room 103b respectively disposed on different floors. The second alarm unit 22 includes second alarm lights 22a, 22b, and 22c respectively disposed in the first therapy monitoring room 103a and the second therapy monitoring room 103b. The second alarm lights 22a and 22b are respectively disposed corresponding to two horizontal therapy rooms 101a. The second alarm lights 22a, 22b, and 22c output light of three colors, for example, red, yellow, and green. That the green light is always-on indicates that the medical facility 1 is currently in a standby safe state. That the yellow light is always-on indicates that a therapy room 101 corresponding to the therapy monitoring room 103 is emitting a beam, and personnel in the room is prompted to pay attention. That the red light is always-on indicates that the current medical facility 1 is in an abnormal state, and all personnel need to evacuate. In another embodiment, the second alarm unit 22 further includes a speaker, configured to broadcast content of the alarm signals, where the content of the alarm signals may include content for indicating safe, attention, or evacuation, and the content of the alarm signals may further include prompting a state of the medical facility 1 or a state that the medical facility is about to enter.

Further, before the therapy room 101 in this embodiment is going to perform irradiation therapy, the first alarm unit 21, the second alarm unit 22, and the third alarm unit 23 all output beam-emitting notification signals, and particularly, may output the signals through the speaker, to prompt that the current therapy room 101 is about to start irradiation. After the therapy room 101 in this embodiment starts the irradiation therapy, the second alarm unit 22 outputs a notification signal indicating that beam emission has been completed, and particularly, may output the signal through the speaker, to prompt that the current therapy room 101 has already emitted the beam, and is performing irradiation therapy. The notification signal indicating that a beam is about to be emitted or indicating that the beam has been emitted may be implemented by operating personnel by inputting a corresponding instruction or by transmitting a corresponding instruction to the alarm unit 20 through the server 30. When there is a therapy room that is currently performing irradiation therapy in all therapy rooms 101, in other words, when the medical facility 1 is in the second state, the second alarm unit 22 disposed in the therapy monitoring room 103 sends an attention signal.

In this embodiment, at least three forms of alarm signals outputted by the third alarm unit 23 are different signals of a same type. In another embodiment, the at least three forms of alarm signals may alternatively be different types of signals, provided that there are apparently differences. This is not limited herein. As shown in FIG. 3, the third alarm unit 23 includes third alarm lights 23a, 23b, 23c, 23d, 23e, 23f, and 23g disposed in an aisle or another room near the therapy room 101. The third alarm light is particularly disposed near the horizontal irradiation room 101a and the vertical irradiation room 101b. The third alarm lights 23a, 23b, 23c, 23d, 23e, 23f, and 23g output light of two colors, for example, green and yellow. One green light may be disposed, and a quantity of yellow lights corresponds to a quantity of therapy rooms 101. That the green light is always-on indicates that the medical facility 1 is currently in a standby safe state. The yellow lights are in a one-to-one correspondence with the therapy rooms 101. When one of the therapy rooms 101 is emitting a beam to perform irradiation therapy, a yellow light of the third alarm light 23a corresponding to the therapy room 101 is always-on, to remind related personnel to pay attention, and yellow lights corresponding to therapy rooms not in a beam emitting state is off. When lights of all colors of the third alarm lights 23a, 23b, 23c, 23d, 23e, 23f, and 23g are all flashing, it indicates that the medical facility 1 is in an abnormal state, and all personnel need to evacuate. In another embodiment, the third alarm unit 23 further includes a speaker, configured to broadcast content of the alarm signals, where the content of the alarm signals may include content for indicating safe, attention, or evacuation, and the content of the alarm signals may further include prompting a state of the medical facility 1 or a state that the medical facility is about to enter. As shown in FIG. 3, the third alarm unit 23 further includes fourth alarm lights 23h, 23i, 23j, 23k, and 231 disposed relatively far away from the therapy room 101. It may be understood that, apart from the third alarm lights disposed near the therapy room 101, the fourth alarm lights are disposed in other parts of the non-therapy area. The fourth alarm lights output light of two colors, for example, red and green. When the green light is always-on, it indicates that the medical facility 1 is currently in a standby safe state. When the red light is always-on, it indicates that the medical facility 1 is in an abnormal state, and all personnel need to evacuate.

Further, evacuation signals sent by the first alarm unit 21, the second alarm unit 22, and the third alarm unit 23 may be signals of a type different from those of the safe signal or the attention signal, so that the third state can be apparently different from the first state and the second state. For example, in this embodiment, the alarm unit includes an alarm light, the safe signal or the attention signal is displayed by a light of a different color being always-on, and the evacuation signal is displayed by a light flickering effect or a light of a color different from those of the safety signal and the attention signal being always-on.

The therapy room, the therapy monitoring room, and the non-therapy area include at least one shielding space, and a shielding door 110 for shielding radiation is disposed in the shielding space. In this embodiment, the shielding space is surrounded by a wall having a ray shielding function, for example, a cement partition wall having a neutron shielding capability. Specifically, the shielding door 110 is disposed at a communication place between the therapy room 101 and another room or area, the shielding door 110 may be disposed at a communication place between the accelerator room 102a and another area; or the shielding door 110 may be disposed at a communication place between the beam conversion and storage room and another area. A fourth alarm unit 24 is disposed on the shielding door 110, and the fourth alarm unit 24 outputs alarm signals of at least three forms. The alarm signals outputted by the fourth alarm unit 24 includes a fifth alarm light (not shown in the figure) disposed at a door frame of the shielding door 110. The fifth alarm light outputs light of three colors. The first color, for example, green, is a safe signal for indicating that the medical facility 1 is in a standby safe state. The second color, for example, yellow, is an attention signal for indicating that the current shielding space is in a beam-emitting state. The third color, for example, red, is an evacuation signal for indicating that the medical facility 1 is in an abnormal state.

The server 30 further includes a display device 33, a client host 31, and a server host 32. The server 30 may be set up based on micro-controller units (MCUs). The client host 31 is connected to the therapy system 200, and is configured to: receive input of a user, store and process data of the therapy system 200 and the radiation safety control system 100, control operating of the therapy system 200 in the medical facility 1, and the like. The display device 33 is connected to the server host 32 and the client host 31, and is configured to display data information related to the therapy system 200 and the radiation safety control system 100, including an operating parameter of the medical facility 1, an environmental radiation amount detected by the radiation detection apparatus 10, an alarm situation of the alarm apparatus 20, and the like. The server host 32 is connected to the display device 33 and the client host 31 through a bus, and summarizes all data information. Further, in an embodiment, the client host 31 and the display device 33 are disposed in the therapy monitoring room 103, and each of the horizontal irradiation room 101a and the vertical irradiation room 101b has a corresponding client host 31 and display device 33.

In another embodiment, the radiation safety control system 100 in the foregoing embodiment may be further configured to monitor safety of another radiation therapy system, such as a proton therapy system or a heavy ion therapy system. The radiation safety control system 100 may further be configured to monitor safety of a diagnostic medical device, such as an X-ray diagnosis system or a PRT/CT diagnosis system. Correspondingly, the radiation detection apparatus 10 detects an environmental radiation amount of X-rays or other radiation particles in the medical facility 1.

Another embodiment of the present invention further provides a control method for a radiation safety control system of a medical facility, including the following steps:
S100: Detect an environmental radiation amount in real time.
S200: Compare the environmental radiation amount detected in real time with a preset threshold in a server, and determine a state of the medical facility.
S300: An alarm unit sends a signal based on the state of the medical facility.

Further, the control method further includes the following steps:
S201: Comparing the environmental radiation amount detected in real time with the threshold. When the environmental radiation amount is less than the threshold, and no radiation is generated in the medical facility, the medical facility is in a first state; when the environmental radiation amount is less than the threshold, and the medical facility includes at least one area in which radiation irradiation therapy is performed, the medical facility is in a second state; and when the environmental radiation amount is greater than or equal to the threshold, the medical facility is in a third state.
S301: When the medical facility is in the first state, an alarm unit in the facility sends a safe signal; when the medical facility is in the second state, an alarm unit in an area in which radiation irradiation therapy is performed sends an evacuation signal, and an alarm unit in another area sends an attention signal; and when the medical facility is in the third state, an alarm unit in the facility sends an evacuation signal.

It should be understood that, although the steps are displayed sequentially according to the instructions of the arrows in the flowcharts of the foregoing embodiments, these steps are not necessarily performed sequentially according to the sequence instructed by the arrows. Unless explicitly described in the specification, execution of the steps is not strictly limited, and the steps may be performed in other sequences. In addition, at least a part of the steps in the flowcharts of the foregoing embodiments may include a plurality of steps or a plurality of stages. These steps or stages are not necessarily performed and completed at the same time, and may be performed at different times. Besides, these operations or stages may be not necessarily performed sequentially, and may be performed in turn or alternately with other steps or at least a part of operations or stages in other steps.

A person of ordinary skill in the art should understand that all or a part of the processes of the method in the foregoing embodiments may be implemented by a computer program instructing relevant hardware. The computer program may be stored in a non-volatile computer-readable storage medium. When the computer program is executed, the processes of the method in the foregoing embodiments are performed. Any reference to a storage module, a database, or another medium used in the various embodiments provided in this application may include at least one of a non-volatile memory or a volatile memory. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, or the like. The volatile memory may include a random access memory (RAM), an external cache, or the like. For illustration rather than limitation, the RAM may be in various forms, for example, may be a static random access memory (SRAM) or a dynamic random access memory (DRAM). The database involved in the embodiments provided in this application may include at least one of a relational database and a non-relational database. The non-relational database may include a blockchain-based distributed database or the like, but is not limited thereto. The processor involved in the embodiments provided in this application may be a general-purpose processor, a central processing unit, a graphics processor, a digital signal processor, a programmable logic device, a quantum computing-based data processing logic device, or the like, but is not limited thereto.

The technical features in the foregoing embodiments may be combined in different manners to form other embodiments. For concise description, not all possible combinations of the technical features in the embodiment are described. However, the combinations of the technical features are all to be considered as falling within the scope described in this specification provided that they do not conflict with each other.

The foregoing embodiments only show several implementations of the present invention and are described in detail, but they are not to be construed as a limit to the patent scope of this application. It should be noted that, a person of ordinary skill in the art may make various changes and improvements without departing from the ideas of the present invention, which shall all fall within the protection scope of the present invention. Therefore, the patent protection scope of the present invention is subject to the protection scope of the appended claims.

## Claims

1. A radiation safety control system of a medical facility, wherein the medical facility comprises a therapy system, the therapy system comprises a radiation generation unit and an irradiation unit, and the radiation generation unit generates a radiation beam, and outputs the radiation beam through the irradiation unit for irradiation; and the radiation safety control system comprises:
a radiation detection apparatus, disposed in the medical facility, and configured to collect an environmental radiation amount of each preset area in the medical facility;
an alarm apparatus, disposed in the medical facility, and performing corresponding alert and prompt based on the collected environmental radiation amount; and
a server, connected to the radiation detection apparatus, and receiving a data value collected by the radiation detection apparatus; the server being connected to the alarm apparatus, and outputting an alarm instruction; and the server being connected to the therapy system, and controlling operating of the therapy system; and
the medical facility comprises at least a first state, a second state, and a third state; when no radiation is generated in the medical facility, the medical facility is in the first state; when the medical facility performs radiation irradiation in at least one preset area in the medical facility, and the environmental radiation amount is less than a preset threshold, the medical facility is in the second state; and when the environmental radiation amount in at least one preset area is greater than or equal to the preset threshold, the medical facility is in the third state.

2. The radiation safety control system of a medical facility according to claim 1, wherein the preset area comprises a therapy room and a therapy monitoring room corresponding to the therapy room; and the alarm apparatus comprises:
a first alarm unit, disposed in the therapy room, and outputting alarm signals of at least two forms; and
a second alarm unit, disposed in the therapy monitoring room, and outputting alarm signals of at least three forms.

3. The radiation safety control system of a medical facility according to claim 2, wherein when the medical facility is in the first state, both the first alarm unit and the second alarm unit send safe signals; when at least one therapy room is in the second state, a first alarm unit in the therapy room in the second state sends an evacuation signal, a second alarm unit in a corresponding therapy monitoring room sends an evacuation signal, and a second alarm unit in another therapy monitoring room sends an attention signal; and when the medical facility is in the third state, both the first alarm unit and the second alarm unit send evacuation signals.

4. The radiation safety control system of a medical facility according to claim 2, wherein the preset area further comprises a non-therapy area; the alarm apparatus comprises a third alarm unit disposed in the non-therapy area and outputting alarm signals of at least two forms; and when the medical facility is in the first state, the third alarm unit sends a safe signal; when the medical facility is in the third state, the third alarm unit sends an evacuation signal; or when the medical facility is in the second state, the third alarm unit sends an attention signal indicating a status of the therapy room.

5. The radiation safety control system of a medical facility according to claim 2, wherein the alarm apparatus comprises an alarm light and/or a speaker for outputting different information.

6. The radiation safety control system of a medical facility according to claim 5, wherein the alarm light comprises at least two colors, and an operating status of the alarm light comprises at least always-on, flickering, or off.

7. The radiation safety control system of a medical facility according to claim 5, wherein the preset area comprises at least one therapy room; the speaker outputs a beam-emitting notification signal before any one of the therapy room performs irradiation; and the speaker outputs a voice notification after any one of the therapy room starts irradiation, to prompt personnel in the medical facility that the irradiation unit is operating.

8. The radiation safety control system of a medical facility according to claim 4, wherein the therapy room, the therapy monitoring room, and the non-therapy area comprise at least one shielding space, a shielding door for shielding radiation is disposed in the shielding space, a fourth alarm unit is disposed on the shielding door, and the fourth alarm unit outputs alarm signals of at least three forms.

9. The radiation safety control system of a medical facility according to claim 8, wherein when the medical facility is in the first state, the fourth alarm unit outputs a safe signal; when at least one of the shielding space is in the second state, a fourth alarm unit disposed on a shielding door in the shielding space in the second state outputs an attention signal; and when the medical facility is in the third state, the fourth alarm unit outputs an evacuation signal.

10. The radiation safety control system of a medical facility according to claim 1, wherein the therapy system is a neutron capture therapy system, and the radiation detection apparatus detects an environmental radiation amount of neurons or γ rays in the medical facility.

11. The radiation safety control system of a medical facility according to claim 1, wherein the radiation safety control system further comprises a display device, and the display device is connected to the server, and is configured to display an operating parameter of the medical facility and an alarm status of the alarm apparatus.

12. The radiation safety control system of a medical facility according to claim 11, wherein the radiation safety control system further comprises a client host and a server host; the client host is connected to the therapy system, and is configured to: store and process data of the therapy system and the radiation safety control system, and control running of the therapy system; and the server host is connected to the display device and the client host.

13. A control method for a radiation safety control system of a medical facility, comprising the following steps:
detecting an environmental radiation amount in real time by using a radiation detection apparatus;
comparing the environmental radiation amount detected in real time with a preset threshold in a server, and determining a state of the medical facility; and
controlling, by the server based on the state of the medical facility, an alarm apparatus to output an alarm signal.

14. The control method for a radiation safety control system of a medical facility according to claim 13, wherein comparing the environmental radiation amount detected in real time with the threshold, when no radiation is generated in the medical facility, the medical facility is in a first state; when the environmental radiation amount is less than the threshold, and the medical facility comprises at least one area in which radiation irradiation is performed, the area in which radiation irradiation is performed is in a second state; and when the environmental radiation amount is greater than or equal to the threshold, the medical facility is in a third state.

15. The control method for a radiation safety control system of a medical facility according to claim 14, wherein when the medical facility is in the first state, the alarm apparatus in the medical facility sends a safe signal; when the medical facility is in the second state, an alarm apparatus in the area in which radiation irradiation is performed sends an evacuation signal, and an alarm apparatus in another area in which radiation irradiation is not performed sends an attention signal; and when the medical facility is in the third state, the alarm apparatus in the medical facility sends an evacuation signal.
